(19) Europäisches Patentamt
European Patent Office
Office européen des brevets

(11) Numéro de publication : **0 018 927**
**B1**

# (12) FASCICULE DE BREVET EUROPÉEN

(45) Date de publication du fascicule du brevet : **24.02.82**

(51) Int. Cl.³ : **C 07 C 51/12**, C 07 C 53/08, C 07 C 53/122, C 07 C 57/32

(21) Numéro de dépôt : **80420045.9**

(22) Date de dépôt : **23.04.80**

## (54) Procédé de préparation d'acides carboxyliques par carbonylation.

(30) Priorité : **25.04.79 FR 7911209**

(43) Date de publication de la demande : **12.11.80 (Bulletin 80/23)**

(45) Mention de la délivrance du brevet : **24.02.82 Bulletin 82/08**

(84) Etats contractants désignés : **AT BE CH DE GB IT LI LU NL SE**

(56) Documents cités :
**DE - A - 2 847 241**
**CHEMICAL ABSTRACTS, vol. 77, n° 1 3. juillet 1972, réf. 4923r, page 438 COLUMBUS, OHIO (US)**

(73) Titulaire : **RHONE-POULENC INDUSTRIES**
**Brevets Pharma 25 Quai Paul Doumer**
**F-92408 Courbevoie Cedex (FR)**

(72) Inventeur : **Gauthier-Lafaye, Jean**
**21, rue Duguesclin**
**F-69006 Lyon (FR)**
Inventeur : **Perron, Robert**
**La Pecollère**
**F-69390 Charly (FR)**

(74) Mandataire : **Varniere-Grange, Monique et al**
**RHONE-POULENC INDUSTRIES Centre de Recherches des Carrières Service Brevets**
**F-69190 Saint Fons (FR)**

**EP 0 018 927 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation d'acides carboxyliques par carbonylation

La présente invention a pour objet un procédé de préparation d'acides carboxyliques, et plus particulièrement de l'acide acétique, par carbonylation d'un alcool.

Il est bien connu, en particulier, que l'acide acétique peut être produit par carbonylation du méthanol, dans des conditions relativement sévères de pression, en présence de nickel et d'un halogène libre ou combiné.

C'est ainsi qu'il a été proposé, notamment, (Cf. Brevet des Etats-Unis d'Amérique n° 2 729 651) de réaliser la carbonylation du méthanol en présence de complexes du nickel, obtenus par réaction d'halogénures de nickel avec des halogénures d'ammonium (ou de phosphonium) quaternaire, complexes de formule générale

$$\left[A_4\ M\right]_2\ Ni\ X_4$$

dans laquelle X représente un atome de brome ou d'iode, M un atome de phosphore ou d'azote, A étant, par exemple, un radical alcoyle inférieur.

Ces complexes peuvent être engagés sous cette forme dans la réaction en cause, ou bien ils peuvent être formés in situ. Toutefois, bien que la pression mise en œuvre lors de la réaction de carbonylation soit élevée (de l'ordre de 700 atmosphères) l'efficacité du système catalytique, exprimée en terme de productivité horaire, est très faible.

Cette productivité exprimée soit par rapport au volume réactionnel, soit par rapport à la masse de nickel mise en œuvre, a pu être sensiblement améliorée en engageant dans la réaction en cause d'une part, un halogénure de nickel et d'autre part, un halogénure d'ammonium (ou de phosphonium) quaternaire en quantité supérieure à celle requise par la stœchiométrie de formation des complexes de formule rappelée ci-avant (Cf. Brevet allemand 933.148). Toutefois, même dans ce dernier cas, les conditions de pression restent sévères.

Plus récemment, il a été proposé des systèmes catalytiques permettant de carbonyler le méthanol dans des conditions moins sévères de pression. Ainsi, la demande de brevet français 2. 370. 023 décrit la carbonylation du méthanol en présence d'au moins 10 moles d'iodure de méthyle pour 100 moles de méthanol, de nickel, et d'une phosphine libre et/ou complexée avec le nickel, sous une pression inférieure à 70 bars. Néanmoins, l'efficacité d'un tel système, exprimée en terme de productivité horaire reste faible. En outre, l'intérêt industriel d'un tel procédé est compromis par l'instabilité et le coût des phosphines nécessaires à sa mise en œuvre ainsi que par les risques élevés de corrosion des installations en raison de la présence indispensable de très grandes quantités d'iodure de méthyle.

Il a maintenant été trouvé qu'il est possible de préparer des acides carboxyliques, en particulier des acides alcanoïques inférieurs, et notamment l'acide acétique par carbonylation d'alcools en présence de nickel et d'au moins un halogénure d'alkyle avec une productivité accrue, dans ces conditions de pression relativement peu sévères, tout en obviant aux inconvénients précités.

La présente invention a donc pour objet un procédé perfectionné de carbonylation d'alcools sous une pression totale au plus égale à 150 bars, en présence d'une quantité efficace de nickel, d'au moins un halogénure alcalin ou alcalino-terreux, d'au moins un halogénure d'alkyle et d'un solvant choisi dans le groupe constitué par la tétraméthylènesulfone, ses dérivés obtenus par remplacement d'un ou plusieurs atomes d'hydrogène au moyen de radicaux alkyles renfermant de 1 à 4 atomes de carbone, les éthers alkyliques de polyéthylène glycols de formule

$$R^1—O—(CH_2—CH_2—O)_m—R^2 \qquad (I)$$

dans laquelle $R^1$ et $R^2$, identiques ou différents, représentent un radical alkyle renfermant de 1 à 4 atomes de carbone, et m est un entier pouvant varier de 1 à 8 ($1 \leq m \leq 8$), et les composés de formule

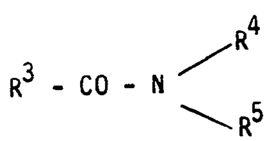

(II)

dans laquelle $R^3$, $R^4$ et $R^5$, identiques ou différents, représentent un radical alkyle linéaire ou ramifié, un radical cycloalkyle, un radical aralkyle ou un radical aryle monocyclique, ayant au plus 10 atomes de carbone, deux des radicaux $R^3$, $R^4$ ou $R^5$ pouvant constituer ensemble un radical unique divalent $—(CH_2)_n—$, n étant un entier compris entre 3 et 12 ($3 \leq n \leq 12$), $R^3$ pouvant en outre représenter l'hydrogène ou un radical

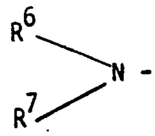

dans lequel $R^6$ et $R^7$, identiques ou différents, représentent un radical alkyle ayant de 1 à 4 atomes de carbone.

Il a été trouvé de manière surprenante qu'un tel système permet d'opérer à des températures supérieures à 150 °C et par suite d'atteindre des productivités horaires élevées, de supprimer la période d'initiation de la réaction, de travailler sous une faible pression partielle de monoxyde de carbone avec une quantité restreinte d'halogénure d'alkyle et ainsi de limiter la corrosion de l'appareillage, tout en facilitant le recyclage de la combinaison catalytique.

Il a été trouvé de manière inattendue, en particulier dans le cadre de la carbonylation du méthanol en acide acétique, que les résultats ne sont pas satisfaisants si la pression partielle du monoxyde de carbone est trop élevée ; ceci est d'autant plus surprenant que de nombreux travaux antérieurs avaient mis en évidence la nécessité de travailler sous des pressions très élevées.

Selon la présente invention, on fait réagir de l'oxyde de carbone sur un alcool, notamment sur un alcool alkylique inférieur, pour produire l'acide carboxylique correspondant, c'est-à-dire renfermant un atome de carbone de plus dans la molécule que l'alcool de départ selon le schéma réactionnel ci-après :

$$ROH + CO \longrightarrow RCOOH$$

dans lequel R représente un radical alkyle linéaire, ramifié ou cyclique ayant de 1 à 11 atomes de carbone ou un radical $\emptyset—C_pH_{2p}—$ dans lequel p est un entier compris entre 1 et 6 ($1 \leq p \leq 6$), ce radical R pouvant porter un ou plusieurs substituants, inertes dans les conditions de réaction. De préférence, R représente un radical alkyle inférieur linéaire ou ramifié ayant de 1 à 4 atomes de carbone, et plus particulièrement un radical méthyle.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en œuvre du présent procédé, on peut citer : le carbonate, l'oxyde, l'hydroxyde, les halogénures, en particulier l'iodure, les carboxylates, en particulier l'acétate, de nickel. Le nickel carbonyle convient également bien. On utilise de préférence le nickel RANEY, l'iodure de nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable. De manière générale, une quantité comprise entre 5 et 2 000 milliatomes-grammes de nickel par litre de solution conduit à des résultats satisfaisants. On opère de préférence avec une teneur comprise entre 20 et 1 000 milliatomes-grammes de nickel par litre.

Le procédé selon la présente invention nécessite également la présence d'au moins un halogénure alcalin ou alcalino-terreux. Les chlorures, bromures et iodures alcalins ou alcalino-terreux peuvent être utilisés dans le cadre du présent procédé. On utilise, de préférence, les iodures alcalins ou alcalino-terreux, et de manière particulièrement avantageuse l'iodure de sodium ou de potassium.

La quantité d'halogénure alcalin ou alcalino-terreux à mettre en œuvre peut varier dans de larges limites. En général, une teneur comprise entre 0,1 et 20 ions-grammes d'halogénure $X^-$ par atome-gramme de nickel présent dans le milieu réactionnel conduit à des résultats satisfaisants. Toutefois cette teneur est, de préférence, comprise entre 1 et 10 ions-grammes d'halogénure $X^-$, par atome-gramme de nickel.

La mise en œuvre du présent procédé implique, en outre, la présence d'au moins un composé de formule R'X dans laquelle X représente un atome de chlore, de brome ou, de préférence, un atome d'iode et R' à la signification donnée pour R, R et R' pouvant être identiques ou différents. Bien entendu l'halogénure d'alkyle qui peut être engagé initialement dans le milieu réactionnel, est susceptible d'être formé in situ à partir de dérivés halogénés tels que $Cl_2$, $Br_2$, $I_2$, HCl, HBr, HI, $NiBr_2$ et $NiI_2$, et de l'alcool (matière de départ). En d'autres termes une partie ou la totalité de l'halogénure d'alkyle nécessaire à la mise en œuvre du présent procédé peut être formée à partir de ses « précurseurs » définis ci-avant.

On constatera en outre que lorsque le dérivé halogéné est choisi parmi les composés du nickel, il peut être considéré comme précurseur non seulement de l'halogénure d'alkyle mais encore comme précurseur du catalyseur métallique. Dans ce cas particulier il s'avère préférable de charger en outre, initialement, un halogénure d'alkyle et/ou un précurseur distinct des halogénures de nickel en cause. L'invention envisage notamment l'utilisation des chlorures, bromures et iodures d'alkyles inférieurs ayant de 1 à 4 atomes de carbone, tels que le bromure et l'iodure de méthyle, le bromure et l'iodure d'éthyle et l'iodure de butyle. On utilise de préférence l'iodure de méthyle, l'iode ou l'acide iodhydrique.

Une teneur en promoteur halogéné comprise entre 0,5 et 20 moles (calculée en halogène X) par

atome-gramme de nickel présent dans le milieu réactionnel est généralement satisfaisante. Selon une variante préférée du présent procédé on opère en présence de 1 à 10 moles (calculées en halogène X) de promoteur halogéné pour un atome-gramme de nickel.

L'une des caractéristiques essentielles de la présente invention réside dans l'utilisation d'un solvant choisi dans le groupe constitué par la tétraméthylènesulfone, ses dérivés obtenus par remplacement d'un ou plusieurs atomes d'hydrogène au moyen de radicaux alkyles renfermant de 1 à 4 atomes de carbone, les éthers alkyliques de polyéthylène glycols de formule :

$$R^1—O—(CH_2—CH_2—O)_m—R^2 \qquad (I)$$

dans laquelle $R^1$ et $R^2$, identiques ou différents, représentent un radical alkyle renfermant de 1 à 4 atomes de carbone, et m est un entier pouvant varier de 1 à 8 ($1 \leq m \leq 8$), et les composés de formule

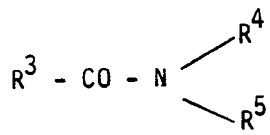

(II)

dans laquelle $R^3$, $R^4$, $R^5$, identiques ou différents, représentent un radical alkyle linéaire ou ramifié, un radical cycloalkyle, un radical aralkyle ou un radical aryle monocyclique, ayant au plus 10 atomes de carbone, deux des radicaux $R^3$, $R^4$ ou $R^5$ pouvant constituer ensemble un radical unique divalent $—(CH_2)_n—$, n étant un entier compris entre 3 et 12 ($3 \leq n \leq 12$), $R^3$ pouvant en outre représenter l'hydrogène ou un radical

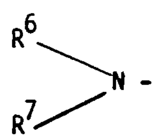

dans lequel $R^6$ et $R^7$, identiques ou différents, représentent un radical alkyle ayant de 1 à 4 atomes de carbone.

Ainsi une première catégorie de solvants convenant à la mise en œuvre du présent procédé est constituée par la tétraméthylènesulfone et ses dérivés obtenus par remplacement d'un ou plusieurs atomes d'hydrogène au moyen de radicaux alkyles inférieurs, ayant de 1 à 4 atomes de carbone, en particulier la méthyl-3-tétraméthylènesulfone et la diméthyl-2,4-tétraméthylènesulfone.

Une autre classe de solvants convenant également à la mise en œuvre de la présente invention est constituée par les éthers alkyliques de polyéthylèneglycols de formule

$$R^1—O—(CH_2CH_2O)_m—R^2 \qquad (I)$$

dans laquelle $R^1$, $R^2$ et m ont la signification donnée ci-avant.

A titre d'exemples d'éthers alkyliques de polyéthylèneglycols utilisables dans le cadre du présent procédé, on peut citer : l'éther diméthylique du diéthylèneglycol, l'éther diéthylique du diéthylèneglycol, l'éther dibutylique du diéthylèneglycol, l'éther éthylique-butylique du diéthylèneglycol, l'éther diméthylique du triéthylèneglycol, l'éther diméthylique du tétraéthylèneglycol, communément appelé tétraglyme. La tétraglyme convient particulièrement bien à la mise en œuvre du présent procédé.

Une classe de solvants qui s'avèrent particulièrement avantageux dans le cadre de la présente invention est constituée par les composés de formule

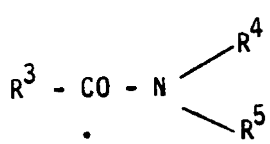

(II)

dans laquelle $R^3$, $R^4$ et $R^5$ ont la signification donnée précédemment.

A titre d'exemples de tels composés, on peut citer : la tétraméthylurée, le N,N-diméthylformamide, le N-N-diéthylformamide, le N,N-dibutylformamide, le N,N-diméthylacétamide, le N,N-diéthylacétamide, le N,N-dicyclohexylacétamide, le N,N-diméthylpropionamide, le N,N-diéthylpropionamide, le N,N-dicyclohexylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la N-acétylpipéridine, la N-n-butyrylpipéridine, la N-méthylpyrrolidone-2, la N-éthylpyrrolidone-2, la N-méthylpipéridone-2, le N-méthyl-caprolactame.

Les composés de formule (II) dans laquelle $R^3$, $R^4$ et $R^5$, identiques ou différents représentent un radical alkyle linéaire ou ramifié, un radical cycloalkyle, un radical aralkyle ou un radical aryle monocyclique, ayant au plus 10 atomes de carbone, les radicaux $R^3$ et $R^4$ (ou $R^5$) pouvant constituer ensemble un radical unique divalent $-(CH_2)_n-$, n ayant la signification donnée précédemment, $R_1$ pouvant en outre représenter l'hydrogène, s'avèrent particulièrement avantageux.

Selon une variante avantageuse du procédé selon l'invention, on opère en présence de N-méthylpyrrolidone-2.

De la quantité précise de solvant mise en œuvre dépendra, pour une large part, la vitesse de réaction. De manière générale des résultats satisfaisants sont obtenus quand le solvant représente au moins 20 % en volume de l'alcool engagé à la réaction. L'homme de l'art sera à même d'ajuster la quantité de solvant en fonction de la nature précise de l'alcool à carbonyler, de la nature du solvant choisi et des autres paramètres de la réaction pour atteindre la vitesse de réaction souhaitée.

Ainsi dans le cas où l'on prépare l'acide acétique par carbonylation du méthanol en présence de N-méthylpyrrolidone, la N-méthylpyrrolidone représente, de préférence, au moins 40 % en volume du méthanol.

Une température de réaction d'au moins 100 °C, est généralement requise pour obtenir une vitesse de réaction suffisante. Une gamme de température allant de 120 à 240 °C est satisfaisante. De préférence, la température est comprise entre 150 et 220 °C.

Le procédé de carbonylation selon la présente invention est conduit en phase liquide.

Dans le cadre du présent procédé, il n'est pas nécessaire de purifier ou de sécher l'alcool. On peut utiliser les alcools de qualité technique, renfermant éventuellement de faibles quantités d'eau.

Le procédé de carbonylation selon l'invention est conduit sous une pression supérieure à la pression atmosphérique, la pression partielle du monoxyde de carbone étant toutefois inférieure à 150 bars. On recommande plus particulièrement d'opérer avec une pression partielle de monooxyde de carbone inférieure à 100 bars. De manière particulièrement avantageuse on utilise une pression partielle de monoxyde de carbone comprise entre 2 et 60 bars.

On met en œuvre, de préférence, le monoxyde de carbone sous forme essentiellement pur tel qu'il se présente dans le commerce. Toutefois, la présence d'impuretés telles que, par exemple du dioxyde de carbone, de l'oxygène, du méthane et de l'azote, n'est pas nuisible. La présence d'hydrogène, même en des proportions relativement importantes, n'est pas gênante.

En fin de réaction, on sépare le mélange réactionnel en ses divers constituants, par tout moyen approprié, par exemple par distillation.

Le procédé selon l'invention convient particulièrement bien à la préparation de l'acide acétique par carbonylation du méthanol..

Les exemples ci-après illustrent l'invention sans toutefois en limiter le domaine ou l'esprit. Les conventions utilisées dans ces exemples sont les suivantes :

AcOH désigne l'acide acétique

AcOMe désigne l'acétate de méthyle

Pr désigne la productivité exprimée en grammes d'acide acétique potentiel produit par heure et par litre de volume réactionnel.

## Exemple 1

Dans un autoclave en acier inoxydable Z8-CNDT 17-12 (norme AFNOR) de 250 ml de capacité, on charge :

27 ml de méthanol  
70 ml de N-méthylpyrrolidone  
7,02 g (49 millimoles) d'iodure de méthyle  
12 g (72 millimoles) d'iodure de potassium  
20 milliatomes-grammes de nickel sous forme de nickel tétracarbonyle (2,6 ml).

Après fermeture de l'autoclave, on établit une pression de 45 bars à l'aide d'un mélange $CO/H_2$ : 2/l. L'agitation par un système de va-et-vient est mise en route et l'autoclave est porté à 150 °C, en 20 minutes environ, au moyen d'un four annulaire. La pression dans l'autoclave est alors de 60 bars et elle est maintenue à cette valeur par des recharges de monoxyde de carbone. Après une heure de réaction à la température indiquée (absorption totale de 64 bars de monoxyde de carbone), le chauffage et l'agitation sont arrêtés : l'autoclave est alors refroidi et dégazé.

Le mélange réactionnel résultant est analysé par chromatographie gazeuse, après dilution. On obtient ainsi 8,7 g d'acétate de méthyle et 18,1 g d'acide acétique. La productivité en acide potentiel est de 250 g/h/l.

Essai témoin a

On a reproduit l'essai ci-avant, mais en omettant l'iodure de potassium. On obtient 2,9 g d'acétate de méthyle et 1,2 g d'acide acétique. La productivité en acide potentiel n'est que de 36 g/h/l.

Essai témoin b

On a reproduit l'exemple 1, mais en omettant l'iodure de méthyle et en ne chargeant que 20 ml de méthanol. On n'obtient ni acide acétique, ni acétate de méthyle.

Exemples 2 à 7

On a réalisé une série d'essais en reproduisant l'exemple 1, mais en utilisant divers iodures alcalins ou alcalino-terreux. Les conditions particulières ainsi que les résultats obtenus sont portés dans le tableau 1 ci-après :

TABLEAU 1

| Ex. | Iodure | | Résultats | | |
|---|---|---|---|---|---|
| N° | Nature | m. Mol (I) | AcOMe (g) | AcOH (g) | Pr |
| 2 | LiI | 100 | 8,8 | 17,5 | 245 |
| 3 | K I | 100 | 8,5 | 22,7 | 295 |
| 4 | NaI | 100 | 8,15 | 22,3 | 290 |
| 5 | CsI | 100 | 7,15 | 14,3 | 200 |
| 6 | $CaI_2$ | 173 | 6,15 | 23,8 | 290 |
| 7 | $MgI_2$ (*) | 200 | 6,3 | 9,95 | 150 |

(*) $MgI_2$, $8H_2O$

Exemples 8 à 12

On a réalisé une autre série d'essais en reproduisant l'exemple 1, mais en chargeant 70 ml de divers solvants. Les conditions particulières ainsi que les résultats obtenus sont portés dans le tableau 2 ci-après :

TABLEAU 2

| Ex. N° | Solvant | AcOMe (g) | AcOH (g) |
|---|---|---|---|
| 8 | Tétraméthylènesulfone | 9,4 | 4,2 |
| 9 | Tétraméthylurée | 5,8 | — |
| 10 | Tétraglyme (*) | 14,3 | 10,5 |
| 11 | Diméthylformamide | 4,4 | — |
| 12 | Diméthylacétamide | 12,0 | 8,2 |

(*) Essai réalisé avec 100 m.Mol de KI et pendant 3 h 45 mn.

Exemples 13 à 17

Dans l'autoclave utilisé à l'exemple 1, on a chargé 27 ml de méthanol, 70 ml de solvant, de l'iodure de méthyle, 100 m.Mol d'iodure de sodium, et 20 milliatomes-grammes de nickel sous forme de nickel tétracarbonyle. Après fermeture de l'autoclave on établit une pression de 45 bars à l'aide de monoxyde de carbone. L'agitation est alors mise en route et l'autoclave est porté à 150 °C, en 20 minutes environ. La pression dans l'autoclave est alors de 60 bars et elle est maintenue à cette valeur par des recharges de monoxyde de carbone. La durée de réaction est de 2 heures. Les conditions particulières ainsi que les résultats obtenus sont portés dans le tableau 3 ci-après :

TABLEAU 3

| Ex. N° | m.Mol de $CH_3I$ | Solvant | AcOMe (g) | AcOH (g) |
|---|---|---|---|---|
| 13 | 52 | N,N-Diéthylacétamide | 10,2 | 27,2 |
| 14 | 51 | N-Ethylpyrrolidone | 8,1 | 26,2 |
| 15 | 51 | N,N-Diéthyl-m-toluamide | 14,3 | 10,1 |
| 16 | 50 | N,N-Dibutylformamide | 13,6 | 5,1 |
| 17 | 49 | N-Méthylpipéridone | 9,3 | 12,9 |

Pr = 180 g/h × l dans l'exemple 13 et 165 g/h × l dans l'exemple 14.

Exemple 18

En suivant le mode opératoire décrit pour les exemples 13 à 17 on a chargé 20 milliatomes-grammes de nickel sous forme de $Ni(CO)_4$, 72 m.Mol de KI, 43 m.Mol de $CH_3I$, 27 ml d'éthanol et 70 ml de N-méthylpyrrolidone. La pression initiale du monoxyde de carbone était de 23 bars. La température était de 150 °C, et la pression totale a été maintenue à 40 bars par des recharges de monoxyde de carbone. En 5 heures de réaction on a obtenu 6,3 g d'acide propionique.

Exemple 19

On a reproduit l'exemple 18 en chargeant cette fois-ci 10 milliatomes-grammes de nickel sous forme de $Ni(CO)_4$, 72 m.Mol de KI, 50 m.Mol de $CH_3I$, 27 ml d'alcool benzylique et 70 ml de N-méthylpyrrolidone. En 4 heures de réaction on a obtenu 24,2 g d'acide phénylacétique.

Exemple 20

Dans un autoclave en acier inoxydable Z8-CNDT 17-12 (norme AFNOR) de 250 ml de capacité, on charge 20 ml de méthanol, 80 ml de N-méthylpyrrolidone, 102 m.Mol de NaCl, 35 m.Mol d'iode et 32 m At-g de nickel sous forme de $Ni(OAc)_2$, $4H_2O$. Après fermeture de l'autoclave, on établit une pression de 45 bars à l'aide d'un mélange $CO/H_2$ : 2/l. L'agitation est mise en route et l'autoclave est porté à 180 °C, en 20 minutes environ. La pression dans l'autoclave est maintenue à la valeur de 60 bars par des recharges de CO pur. Après 2 heures de réaction à la température indiquée, on a obtenu 3,6 g d'acétate de méthyle et 20,2 g d'acide acétique.

Exemple 21

Dans un autoclave en acier inoxydable Z8-CNDT 17-12 (norme AFNOR) de 250 ml de capacité, on charge 27 ml de méthanol, 60 ml de N,N-diméthylacétamide, 43 m.Mol de NaBr, 125 m.Mol de $n—C_4H_9I$ et 20 m At-g de nickel sous forme de $Ni(CO)_4$. Après fermeture de l'autoclave, on établit une pression de 40 bars à l'aide de monoxyde de carbone. L'agitation est mise en route et l'autoclave est porté à 170 °C, en 20 minutes environ. La pression dans l'autoclave est maintenue à la valeur de 60 bars par des recharges de CO pur. Après 1 heure 55 minutes de réaction à la température indiquée, on a obtenu 6,8 g d'acétate de méthyle et 19,6 g d'acide acétique. (Pr = 130 g/h × l).

Exemple 22

Dans un autoclave en acier inoxydable Z8-CNDT 17-12 (norme AFNOR) de 250 ml de capacité, on charge 30 ml de méthanol, 70 ml de N-méthylpyrrolidone, 100 m.Mol de NaI, 123 m.Mol de $C_2H_5I$ et 4,2 g de nickel RANEY. Après fermeture de l'autoclave, on établit une pression de 45 bars à l'aide d'un mélange $CO/H_2$ : 2/l. L'agitation est mise en route et l'autoclave est porté à 185 °C, en 20 minutes environ. La pression dans l'autoclave est maintenue à la valeur de 60 bars par des recharges de CO pur. Après 1 heure 5 minutes de réaction à la température indiquée, on a obtenu 1 g d'acétate de méthyle et 28,2 g d'acide acétique. (Pr = 270 g/h × l).

Exemple 23

Dans un autoclave en acier inoxydable Z8-CNDT 17-12 (norme AFNOR) de 250 ml de capacité, on charge 25 ml de méthanol, 60 ml de N,N-diméthylacétamide, 66 m.Mol de NaI, 200 m.Mol de $C_2H_5Br$ et 19 m At-g de nickel sous forme de $NiI_2$. Après fermeture de l'autoclave, on établit une pression de 45 bars à l'aide d'un mélange $CO/H_2$ : 2/l. L'agitation est mise en route et l'autoclave est porté à 165 °C, en 20 minutes environ. La pression dans l'autoclave est maintenue à la valeur de 60 bars par des recharges de CO pur. Après 1 heure 25 minutes de réaction à la température indiquée, on a obtenu 7,5 g d'acétate de méthyle et 15,2 g d'acide acétique. (Pr = 150 g/h × l).

Exemple 24

Dans un autoclave en acier inoxydable Z8-CNDT 17-12 (norme AFNOR) de 250 ml de capacité, on charge 27 ml de méthanol, 70 ml de N-méthylpyrrolidone, 72 m.Mol de KI, 50 m.Mol de $CH_3I$ et 20 m At-g de nickel sous forme de $Ni(CO)_4$. Après fermeture de l'autoclave, on établit une pression de 30 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 4 heures 30 minutes de réaction à la température indiquée, on a obtenu 2,5 g d'acétate de méthyle et 41,2 g d'acide acétique. (Pr = 96 g/h × l).

Exemple 25

Dans un autoclave en acier inoxydable Z8-CNDT 17-12 (norme AFNOR) de 250 ml de capacité, on charge 47 ml de méthanol, 50 ml de N-méthylpyrrolidone, 72 m.Mol de KI, 47 m.Mol de $CH_3I$ et 20 m At-g de nickel sous forme de $Ni(CO)_4$. Après fermeture de l'autoclave, on établit une pression de 25 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 6 heures 30 minutes de réaction à la température indiquée, on a obtenu 14,4 g d'acétate de méthyle et 39 g d'acide acétique.

Exemple 26

Dans un autoclave en acier inoxydable Z8-CNDT 17-12 (norme AFNOR) de 250 ml de capacité, on charge 67 ml de méthanol, 30 ml de N-méthylpyrrolidone, 72 m.Mol de KI, 48 m.Mol de $CH_3I$ et 20 m At-g de nickel sous forme de $Ni(CO)_4$. Après fermeture de l'autoclave, on établit une pression de 25 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 4 heures de réaction à la température indiquée, on a obtenu 10,7 g d'acétate de méthyle et 4,2 g d'acide acétique.

Exemple 27

Dans un autoclave en acier inoxydable Z8-CNDT 17-12 (norme AFNOR) de 250 ml de capacité, on charge 20 ml de méthanol, 70 ml de N-méthylpyrrolidone, 72 m.Mol de KI, 164 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 2 heures de réaction à la température indiquée, on a obtenu 8,0 g d'acétate de méthyle et 29 g d'acide acétique. ($Pr = 180\ g/h \times l$).

Exemple 28

Dans un autoclave en acier inoxydable Z8-CNDT 17-12 (norme AFNOR) de 250 ml de capacité, on charge 29,5 ml de méthanol, 70 ml de N-méthylpyrrolidone, 72 m.Mol de KI, 10 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 2,65 g d'acétate de méthyle et 0,18 g d'acide acétique.

Exemple 29

Dans un autoclave en acier inoxydable Z8-CNDT 17-12 (norme AFNOR) de 250 ml de capacité, on charge 27 ml de méthanol, 70 ml de N-méthylpyrrolidone, 100 m.Mol de KI, 50 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 210 °C, en 20 minutes environ. La pression dans l'autoclave est maintenue à la valeur de 60 bars par des recharges de CO pur. Après 1 heure de réaction à la température indiquée, on a obtenu 0,98 g d'acétate de méthyle et 36,5 g d'acide acétique. ($Pr = 375\ g/h \times l$).

Exemple 30

Dans un autoclave en acier inoxydable Z8-CNDT 17-12 (norme AFNOR) de 250 ml de capacité, on charge 27 ml de méthanol, 70 ml de N-méthylpyrrolidone, 72 m.Mol de KI, 49 m.Mol de $CH_3I$ et 5 m At-g de nickel sous forme de $Ni(CO)_4$. Après fermeture de l'autoclave, on établit une pression de 22 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 5 heures de réaction à la température indiquée, on a obtenu 7,7 g d'acétate de méthyle et 14,7 g d'acide acétique.

Exemple 31

Dans un autoclave en acier inoxydable Z8-CNDT 17-12 (norme AFNOR) de 250 ml de capacité, on charge 27 ml de méthanol, 70 ml de N-méthylpyrrolidone, 51 m.Mol de $CH_3I$, 100 m.Mol de KI et 20 m At-g de nickel sous forme de $Ni(CO)_4$. Après fermeture de l'autoclave, on établit une pression de 45 bars à

l'aide d'un mélange $CO/H_2$ : 2/l. L'agitation est mise en route et l'autoclave est porté à 180 °C, en 20 minutes environ. La pression dans l'autoclave est maintenue à la valeur de 60 bars par des recharges de CO pur. Après 1 heure de réaction à la température indiquée, on a obtenu 5,8 g d'acétate de méthyle et 31,3 g d'acide acétique. (Pr = 360 g/h × l).

Exemple 32

Dans un autoclave en acier inoxydable Z8-CNDT 17-12 (norme AFNOR) de 250 ml de capacité, on charge 27 ml de méthanol, 70 ml de N-méthylpyrrolidone, 49 m.Mol de $CH_3I$, 72 m.Mol de KI et 10 m At-g de nickel sous forme de $Ni(CO)_4$. Après fermeture de l'autoclave, on établit une pression de 25 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 5 heures de réaction à la température indiquée, on a obtenu 4,5 g d'acétate de méthyle et 37,2 g d'acide acétique.

Exemple 33

Dans un autoclave en acier inoxydable Z8-CNDT 17-12 (norme AFNOR) de 250 ml de capacité, on charge 27 ml de méthanol, 70 ml de N-méthylpyrrolidone, 49 m.Mol de $CH_3I$, 100 m.Mol de KI et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 8,5 g d'acétate de méthyle et 22,7 g d'acide acétique. (Pr = 295 g/h × l).

Exemple 34

Dans un autoclave en acier inoxydable Z8-CNDT 17-12 (norme AFNOR) de 250 ml de capacité, on charge 27 ml de méthanol, 70 ml de N-méthylpyrrolidone, 20 m.Mol de KI, 51 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ, La pression dans l'autoclave est maintenue à la valeur de 60 bars par des recharges de CO pur. Après 1 heure de réaction à la température indiquée, on a obtenu 7 g d'acétate de méthyle et 5,5 g d'acide acétique. (Pr = 110 g/h × l).

Exemple 35

Dans un autoclave en acier inoxydable Z8-CNDT 17-12 (norme AFNOR) de 250 ml de capacité, on charge 27 ml de méthanol, 70 ml de N-méthylpyrrolidone, 49 m.Mol de $CH_3I$, 150 m.Mol de KI et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 8,8 g d'acétate de méthyle et 21,4 g d'acide acétique. (Pr = 285 g/h × l).

Exemple 36

Dans un autoclave en acier inoxydable Z8-CNDT 17-12 (norme AFNOR) de 250 ml de capacité, on charge 28,5 ml de méthanol, 70 ml de N-méthylpyrrolidone, 28 m.Mol de $CH_3I$, 72 m.Mol de KI et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 9,95 g d'acétate de méthyle et 3,4 g d'acide acétique. (Pr = 110 g/h × l).

Exemple 37

Dans un autoclave en acier inoxydable Z8-CNDT 17-12 (norme AFNOR) de 250 ml de capacité, on charge 27 ml de méthanol, 70 ml de N-méthylpyrrolidone, 100 m.Mol de KI, 50 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 8,3 g d'acétate de méthyle et 20,2 g d'acide acétique. (Pr = 270 g/h × l).

Exemple 38

Dans un autoclave en acier inoxydable Z8-CNDT 17-12 (norme AFNOR) de 250 ml de capacité, on charge 27 ml de méthanol, 70 ml de N-méthylpyrrolidone, 45 m.Mol de $CH_3I$, 40 m.Mol de KI et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 8,15 g d'acétate de méthyle et 9,9 g d'acide acétique. (Pr = 165 g/h × l).

Exemple 39

Dans un autoclave en acier inoxydable Z8-CNDT 17-12 (norme AFNOR) de 250 ml de capacité, on charge 25 ml de méthanol, 70 ml de N-méthylpyrrolidone, 72 m.Mol de KI, 81 m.Mol de $CH_3I$ et 20 m At-g de nickel sous forme de $Ni(CO)_4$. Après fermeture de l'autoclave, on établit une pression de 45 bars à l'aide d'un mélange $CO/H_2$ : 2/l. L'agitation est mise en route et l'autoclave est porté à 150 °C, en 20 minutes environ. La pression dans l'autoclave est maintenue à la valeur de 60 bars par des recharges de CO pur. Après 1 heure de réaction à la température indiquée, on a obtenu 8,1 g d'acétate de méthyle et 19,9 g d'acide acétique. (Pr = 265 g/h × l).

Exemple 40

Dans un autoclave en tantale, de 225 ml de capacité, on charge 28 ml de méthanol, 64 ml de N-méthylpyrrolidone, 73 m.Mol de NaI, 49 m.Mol de $CH_3I$ et 20 m At-g de nickel sous forme de $Ni(CO)_4$. Après fermeture de l'autoclave, on établit une pression de 23 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 1 heure de réaction à la température indiquée, on a obtenu 10 g d'acétate de méthyle et 11,3 g d'acide acétique. (Pr = 200 g/h × l).

Exemple 41

Dans un autoclave en acier inoxydable Z8-CNDT 17-12 (norme AFNOR) de 250 ml de capacité, on charge 28 ml de méthanol, 64 ml de N-méthylpyrrolidone, 49 m.Mol de $CH_3I$, 73 m.Mol de NaI et 20 m At-g de nickel sous forme de $Ni(CO)_4$. Après fermeture de l'autoclave, on établit une pression de 23 bars à l'aide de monoxyde de carbone. L'agitation est mise en route et l'autoclave est porté à 150 °C, en 20 minutes environ. La pression dans l'autoclave est maintenue à la valeur de 40 bars par des recharges de CO pur. Après 1 heure de réaction à la température indiquée, on a obtenu 8,2 g d'acétate de méthyle et 16,1 g d'acide acétique. (Pr = 220 g/h × l).

Exemples 42 à 45

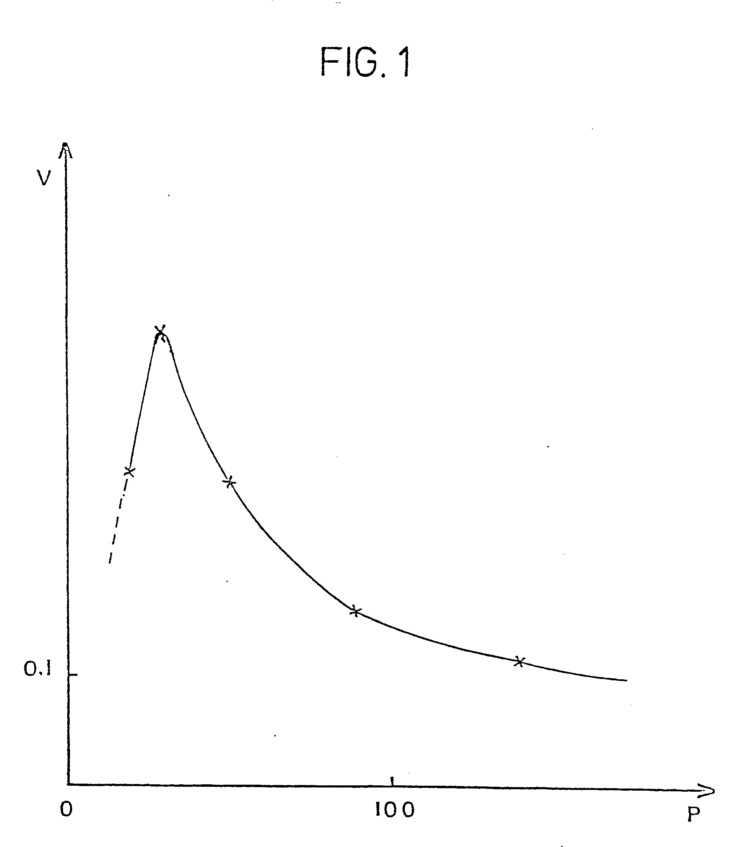

Dans un autoclave en acier inoxydable Z8-CNDT 17-12 (norme AFNOR) de 250 ml de capacité, on a chargé 50 m.Mol de $CH_3I$, 72 m.Mol de KI, 27 ml de méthanol, 70 ml de N-méthylpyrrolidone, et 20 m At-g de nickel sous forme de $Ni(CO)_4$. On a réalisé une série d'essais à 150 °C, avec du monoxyde de carbone sensiblement pur ; la pression partielle du monoxyde de carbone (à la température de réaction) ainsi que les résultats obtenus sont portés dans le tableau 4 ci-après. A partir des résultats portés dans ce tableau, on a tracé le graphique de la figure unique annexée, montrant l'influence de la pression partielle du monoxyde de carbone portée en abscisses et exprimée en bars (P), sur la vitesse de réaction, portée en ordonnées et exprimée en mole de monoxyde de carbone absorbée par heure de réaction (v).

TABLEAU 4

| Ex. N° | P de CO en bars | Durée de réaction | v(Mole/Heure) | Résultats (*) |
|---|---|---|---|---|
| 42 | 20 | 4 h | 0,29 | 91,7 |
| 24 | 30 | 4 h 30 mn | 0,43 | 100 |
| 43 | 50 | 5 h 20 mn | 0,28 | 99 |
| 44 | 90 | 5 h | 0,16 | 72,4 |
| 45 | 140 | 22 h | 0,12 | 100 |

(*) Nombre de moles d'AcOH et d'AcOMe formées, pour 100 moles de méthanol engagées à la réaction.

**Revendications**

1. Procédé de préparation d'un acide monocarboxylique par carbonylation d'un alcool, en phase liquide, à une température comprise entre 120 et 240 °C, en présence d'une quantité efficace de nickel, caractérisé en ce que la réaction est conduite sous une pression partielle de monoxyde de carbone inférieure ou égale à 150 bars, en présence simultanée d'au moins un halogénure alcalin ou alcalino-terreux, d'au moins un halogénure d'alkyle et d'un solvant choisi dans le groupe constitué par la tétraméthylènesulfone, ses dérivés obtenus par remplacement d'un ou plusieurs atomes d'hydrogène au moyen de radicaux alkyles renfermant de 1 à 4 atomes de carbone, les éthers alkyliques de polyéthylène glycols de formule

$$R^1—O—(CH_2—CH_2—O)_m—R^2 \qquad (I)$$

dans laquelle $R^1$ et $R^2$, identiques ou différents, représentent un radical alkyle renfermant de 1 à 4 atomes de carbone, et m est un entier pouvant varier de 1 à 8 ($1 \leq m \leq 8$), et les composés de formule

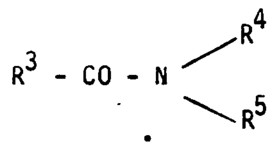
(II)

dans laquelle $R^3$, $R^4$ et $R^5$, identiques ou différents, représentent un radical alkyle linéaire ou ramifié, un radical cycloalkyle, un radical aralkyle ou un radical aryle monocyclique, ayant au plus 10 atomes de carbone, deux des radicaux $R^3$, $R^4$ ou $R^5$ pouvant constituer ensemble un radical unique divalent $—(CH_2)_n—$, n étant un entier compris entre 3 et 12 ($3 \leq n \leq 12$), $R^3$ pouvant en outre représenter l'hydrogène ou un radical

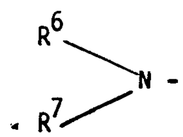

dans lequel $R^6$ et $R^7$, identiques ou différents, représentent un radical alkyle ayant de 1 à 4 atomes de carbone.

2. Procédé de préparation d'un acide monocarboxylique par carbonylation d'un alcool, en phase liquide, à une température comprise entre 120 et 240 °C, en présence d'une quantité efficace de nickel, caractérisé en ce que la réaction est conduite sous une pression partielle de monoxyde de carbone inférieure ou égale à 150 bars, en présence simultanée d'au moins un halogénure alcalin ou alcalino-terreux, d'au moins un halogénure d'alkyle et d'un solvant choisi parmi les composés de formule

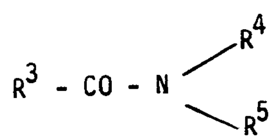
(II)

dans laquelle $R^3$, $R^4$ et $R^5$, identiques ou différents, représentent un radical alkyle linéaire ou ramifié, un radical cycloalkyle, un radical aralkyle ou un radical aryle monocyclique, ayant au plus 10 atomes de carbone, deux des radicaux $R^3$, $R^4$ ou $R^5$ pouvant constituer ensemble un radical unique divalent $—(CH_2)_n—$, n étant un entier compris entre 3 et 12 ($3 \leq n \leq 12$), $R^3$ pouvant en outre représenter l'hydrogène ou un radical

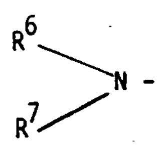

dans lequel $R^6$ et $R^7$, identiques ou différents, représentent un radical alkyle ayant de 1 à 4 atomes de carbone.

3. Procédé selon la revendication 2, caractérisé en ce que le solvant est un composé de formule II dans laquelle $R^3$, $R^4$ et $R^5$, identiques ou différents, représentent un radical alkyle linéaire ou ramifié, un radical cycloalkyle, un radical aralkyle ou un radical aryle monocyclique, ayant au plus 10 atomes de

carbone, deux des radicaux $R^3$, $R^4$ ou $R^5$ pouvant constituer ensemble un radical unique divalent $-(CH_2)_n-$, n étant un entier compris entre 3 et 12 ($3 \leq n \leq 12$).

4. Procédé selon l'une quelconque des revendications précédentes, caractérisé en ce que l'alcool (matière de départ) a pour formule ROH dans laquelle R représente un radical alkyle linéaire, ramifié ou cyclique ayant de 1 à 11 atomes de carbone ou un radical $\Phi-C_pH_{2p}-$ dans lequel p est un entier compris entre 1 et 6 ($1 \leq p \leq 6$), ce radical R pouvant porter un ou plusieurs substituants, inertes dans les conditions de réaction.

5. Procédé selon la revendication 4, caractérisé en ce que R est un radical alkyle ayant de 1 à 4 atomes de carbone.

6. Procédé selon la revendication 5, caractérisé en ce que R est un radical méthyle.

7. Procédé selon l'une quelconque des revendications précédentes, caractérisé en ce que le solvant représente au moins 20 % en volume de l'alcool engagé à la réaction.

8. Procédé selon l'une quelconque des revendications précédentes, caractérisé en ce que la température de réaction est comprise entre 150 et 220 °C.

9. Procédé selon l'une quelconque des revendications précédentes, caractérisé en ce que l'halogénure d'alkyle est un iodure d'alkyle en $C_1$-$C_4$.

10. Procédé selon l'une quelconque des revendications précédentes, caractérisé en ce que l'halogénure d'alkyle est l'iodure de méthyle.

11. Procédé selon l'une quelconque des revendications précédentes, caractérisé en ce que l'halogénure alcalin ou alcalino-terreux est un iodure.

12. Procédé selon l'une quelconque des revendications précédentes, caractérisé en ce que l'halogénure alcalin est choisi parmi les halogénures de sodium et de potassium.

13. Procédé selon l'une quelconque des revendications précédentes, caractérisé en ce que la réaction est conduite en présence de 20 à 1 000 milliatomes-grammes de nickel par litre de solution.

14. Procédé selon l'une quelconque des revendications précédentes, caractérisé en ce que la réaction est conduite en présence de 1 à 10 moles d'halogénure d'alkyle et de 1 à 10 ions-grammes d'halogénure en provenance de l'halogénure alcalin ou alcalino-terreux, par atome-gramme de nickel.

15. Procédé selon l'une quelconque des revendications précédentes, caractérisé en ce que le solvant est la N-méthylpyrrolidone.

16. Procédé selon la revendication 15, caractérisé en ce que le solvant représente au moins 40 % en volume de l'alcool engagé à la réaction.

17. Procédé selon l'une quelconque des revendications précédentes, caractérisé en ce que la pression partielle du monoxyde de carbone est inférieure à 100 bars.

18. Procédé selon la revendication 17, caractérisé en ce que la pression partielle du monoxyde de carbone comprise entre 2 et 60 bars.

**Claims**

1. Process for the preparation of a monocarboxylic acid by carbonylating an alcohol, in the liquid phase, at a temperature between 120 and 240 °C, in the presence of an effective amount of nickel, characterised in that the reaction is carried out under a partial pressure of carbon monoxide which is less than or equal to 150 bars, in the simultaneous presence of at least one alkali metal halide or alkaline earth metal halide, at least one alkyl halide and a solvent chosen from the group comprising tetramethylenesulphone, its derivatives obtained by replacing one or more hydrogen atoms by alkyl radicals containing from 1 to 4 carbon atoms, polyethylene glycol alkyl ethers of the formula

$$R^1-O-(CH_2-CH_2-O)_m-R^2 \qquad (I)$$

in which $R^1$ and $R^2$, which are identical or different, represent an alkyl radical containing from 1 to 4 carbon atoms and m is an integer which can vary from 1 to 8 ($1 \leq m \leq 8$), and the compounds of the formula

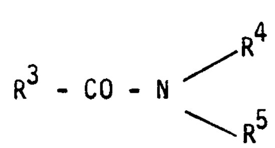

(II)

in which $R^3$, $R^4$ and $R^5$, which are identical or different, represent a linear or branched alkyl radical, a cycloalkyl radical, an aralkyl radical or a monocyclic aryl radical, the said radicals having at most 10 carbon atoms, it being possible for two of the radicals $R^3$, $R^4$ or $R^5$ together to form a single divalent radical $-(CH_2)_n-$, being an integer between 3 and 12 ($3 \leq n \leq 12$), and it also being possible for $R^3$ to represent hydrogen or a radical

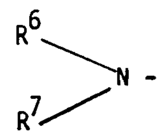

in which $R^6$ and $R^7$, which are identical or different, represent an alkyl radical having from 1 to 4 carbon atoms.

2. Process for the preparation of a monocarboxylic acid by carbonylating an alcohol, in the liquid phase, at a temperature between 120 and 240 °C, in the presence of an effective amount of nickel, characterised in that the reaction is carried out under a partial pressure of carbon monoxide which is less than or equal to 150 bars, in the simultaneous presence of at least one alkali metal halide or alkaline earth metal halide, at least one alkyl halide and a solvent chosen from amongst the compounds of the formula

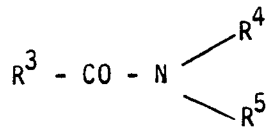

(II)

in which $R^3$, $R^4$ and $R^5$, which are identical or different, represent a linear or branched alkyl radical, a cycloalkyl radical, an aralkyl radical or a monocyclic aryl radical, the said radicals having at most 10 carbon atoms, it being possible for two of the radicals $R^3$, $R^4$ or $R^5$ together to form a single divalent radical $-(CH_2)_n-$, n being an integer between 3 and 12 ($3 \leq n \leq 12$), and it also being possible for $R^3$ to represent hydrogen or a radical

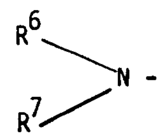

in which $R^6$ and $R^7$, which are identical or different, represent an alkyl radical having from 1 to 4 carbon atoms.

3. Process according to claim 2, characterised in that the solvent is a compound of the formula II, in which $R^3$, $R^4$ and $R^5$, which are identical or different, represent a linear or branched alkyl radical, a cycloalkyl radical, an aralkyl radical or a monocyclic aryl radical, the said radicals having at most 10 carbon atoms, it being possible for two of the radicals $R^3$, $R^4$ or $R^5$ together to form a single divalent radical $-(CH_2)_n-$, n being an integer between 3 and 12 ($3 \leq n \leq 12$).

4. Process according to any one of the preceding claims, characterised in that the alcohol (starting material) has the formula ROH, in which R represents a linear, branched or cyclic alkyl radical having from 1 to 11 carbon atoms, or a radical $\phi-C_pH_{2p}-$, in which p is an integer between 1 and 6 ($1 \leq p \leq 6$), it being possible for this radical R to carry one or more substituents which are inert under the reaction conditions.

5. Process according to claim 4, characterised in that R is an alkyl radical having from 1 to 4 carbon atoms.

6. Process according to claim 5, characterised in that R is a methyl radical.

7. Process according to any one of the preceding claims, characterised in that the solvent represents at least 20 % by volume of the alcohol employed in the reaction.

8. Process according to any one of the preceding claims, characterised in that the reaction temperature is between 150 and 250 °C.

9. Process according to any one of the preceding claims, characterised in that the alkyl halide is a $C_1$-$C_4$ alkyl iodide.

10. Process according to any one of the preceding claims, characterised in that the alkyl halide is methyl iodide.

11. Process according to any one of the preceding claims, characterised in that the alkali metal halide or alkaline earth metal halide is an iodide.

12. Process according to any one of the preceding claims, characterised in that the alkali metal halide is chosen from amongst sodium halides and potassium halides.

13. Process according to any one of the preceding claims, characterised in that the reaction is carried out in the presence of 20 to 1 000 milligram atoms of nickel per litre of solution.

14. Process according to any one of the preceding claims, characterised in that the reaction is carried out in the presence of 1 to 10 mols of alkyl halide and 1 to 10 gram ions of halide originating from the alkali metal halide or alkaline earth metal halide, per gram atom of nickel.

15. Process according to any one of the preceding claims, characterised in that the solvent is N-methylpyrrolidone.

16. Process according to claim 15, characterised in that the solvent represents at least 40 % by volume of the alcohol employed in the reaction.

17. Process according to any one of the preceding claims, characterised in that the partial pressure of the carbon monoxide is less than 100 bars.

18. Process according to claim 17, characterised in that the partial pressure of the carbon monoxide is between 2 and 60 bars.

**Ansprüche**

1. Verfahren zur Herstellung einer Monocarbonsäure durch Carbonylieren eines Alkohols in flüssiger Phase bei einer Temperatur von 120 bis 240 °C in Gegenwart einer wirksamen Menge Nickel, dadurch gekennzeichnet, daß die Reaktion unter einem CO-Partialdruck ≤ 150 bar in Anwesenheit von gleichzeitig mindestens einem Alkali- oder Erdalkalihalogenid, mindestens einem Alkylhalogenid und einem Lösungsmittel ausgeführt wird, das ausgewählt ist aus der Gruppe Tetramethylensulfon und seine Derivate, die durch Ersatz von ein oder mehreren Wasserstoffatomen durch Alkylgruppen mit 1 bis 4 Kohlenstoffatomen im Molekül erhalten worden sind, Polyäthylenglykol-alkyläther der allgemeinen Formel

$$R^1—O—(CH_2—CH_2—O)_m—R^2 \qquad (I)$$

in der $R^1$ und $R^2$ gleich oder verschieden sind und jeweils für eine Alkylgruppe mit 1 bis 4 Kohlenstoffatomen im Molekül stehen und m eine ganze Zahl von 1 bis 8 ist ($1 \leq m \leq 8$), sowie Verbindungen der allgemeinen Formel

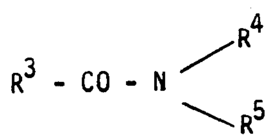

(II)

in der $R^3$, $R^4$ und $R^5$ gleich oder verschieden sind und jeweils für eine lineare oder verzweigte Alkylgruppe, eine Cycloalkylgruppe, eine Aralkylgruppe oder eine monocyclische Arylgruppe, mit höchstens 10 Kohlenstoffatomen im Molekül stehen, zwei der Substituenten $R^3$, $R^4$ oder $R^5$ zusammen eine zweiwertige Gruppe $—(CH_2)_n—$ bilden können, wobei n eine ganze Zahl von 3 bis 12 ist ($3 \leq n \leq 12$) und $R^3$ außerdem Wasserstoff oder eine Gruppe

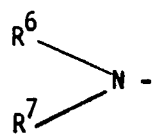

sein kann, in der $R^6$ und $R^7$ gleich oder verschieden sind und jeweils für eine Alkylgruppe mit 1 bis 4 Kohlenstoffatomen stehen.

2. Verharen zur Herstellung einer Monocarbonsäure mittels Carbonylieren eines Alkohols in flüssiger Phase bei einer Temperatur zwischen 120 und 240 °C in Gegenwart einer wirksamen Menge Nickel, dadurch gekennzeichnet, daß die Reaktion unter einem CO-Partialdruck ≤ 150 bar in Anwesenheit von gleichzeitig mindestens einem Alkali- oder Erdalkalihalogenid, mindestens einem Alkylhalogenid und einem Lösungsmittel ausgeführt wird, das ausgewählt wird unter den Verbindungen der allgemeinen Formel

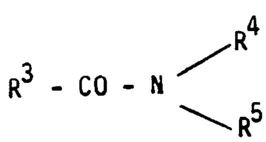

(II)

in der $R^3$, $R^4$ und $R^5$ gleich oder verschieden sein können und jeweils für eine lineare oder verzweigte Alkylgruppe, eine Cycloalkylgruppe, eine Aralkylgruppe oder eine monocyclische Arylgruppe mit höchstens 10 Kohlenstoffatomen stehen, zwei der Substituenten $R^3$, $R^4$ oder $R^5$ zusammen eine zweiwertige Gruppe der allgemeinen Formel $—(CH_2)_n—$ bilden können, in der n eine ganze Zahl von 3 bis 12 ist ($3 \leq n \leq 12$), und $R^3$ außerdem Wasserstoff oder eine Gruppe

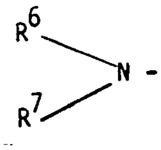

sein kann, in der $R^6$ und $R^7$ gleich oder verschieden sind und jeweils eine Alkylgruppe mit 1 bis 4 Kohlenstoffatomen bedeuten.

3. Verfahren nach Anspruch 2, dadurch gekennzeichnet, daß das Lösungsmittel eine Verbindung der allgemeinen Formel II ist, in der $R^3$, $R^4$ und $R^5$ gleich oder verschieden sind und jeweils für eine lineare oder verzweigte Alkylgruppe, eine Cycloalkylgruppe, eine Aralkylgruppe oder eine monocyclische Arylgruppe, mit höchstens 10 Kohlenstoffatomen stehen oder zwei der Substituenten $R^3$, $R^4$ oder $R^5$ zusammen eine zweiwertige Gruppe der allgemeinen Formel $-(CH_2)_n-$ bedeuten, wobei n eine ganze Zahl von 3 bis 12 ist ($3 \leq n \leq 12$).

4. Verfahren nach einem der vorangegangenen Ansprüche, dadurch gekennzeichnet, daß der als Ausgangsmaterial eingesetzte Alkohol der allgemeinen Formel ROH entspricht, in der R eine lineare, verzweigte oder cyclische Alkylgruppe mit 1 bis 11 Kohlenstoffatomen ist oder eine Gruppe der allgemeinen Formel $C_6H_5-C_pH_{2p}-$, in der p eine ganze Zahl von 1 bis 6 ist ($1 \leq p \leq 6$), wobei die Gruppe R ein oder mehrere Substituenten tragen kann, die unter den Reaktionsbedingungen nicht reagieren.

5. Verfahren nach Anspruch 4, dadurch gekennzeichnet, daß R eine Alkylgruppe mit 1 bis 4 Kohlenstoffatomen ist.

6. Verfahren nach Anspruch 5, dadurch gekennzeichnet, daß R eine Methylgruppe ist.

7. Verfahren nach einem der vorangegangenen Ansprüche, dadurch gekennzeichnet, daß das Lösungsmittel mindestens 20 Vol.-% des eingesetzten Alkohols ausmacht.

8. Verfahren nach einem der vorangegangenen Ansprüche, dadurch gekennzeichnet, daß die Reaktionstemperatur 150 bis 220 °C beträgt.

9. Verfahren nach einem der vorangegangenen Ansprüche, dadurch gekennzeichnet, daß das Alkylhalogenid ein $C_1$-$C_4$-Alkyljodid ist.

10. Verfahren nach einem der vorangegangenen Ansprüche, dadurch gekennzeichnet, daß das Alkylhalogenid Methyljodid ist.

11. Verfahren nach einem der vorangegangenen Ansprüche, dadurch gekennzeichnet, daß das Alkali- oder Erdalkalihalogenid ein Jodid ist.

12. Verfahren nach einem der vorangegangenen Ansprüche, dadurch gekennzeichnet, daß das Alkalihalogenid ein Natriumhalogenid oder Kaliumhalogenid ist.

13. Verfahren nach einem der vorangegangenen Ansprüche, dadurch gekennzeichnet, daß die Reaktion in Gegenwart von 20 bis 1 000 mg-Atom Nickel je Liter Lösung ausgeführt wird.

14. Verfahren nach einem der vorangegangenen Ansprüche, dadurch gekennzeichnet, daß die Reaktion in Gegenwart von 1 bis 10 mol Alkylhalogenid und von 1 bis 10 g-Ion Halogenid aus dem Alkalihalogenid oder Erdalkalihalogenid, je g-Atom Nickel ausgeführt wird.

15. Verfahren nach einem der vorangegangenen Ansprüche, dadurch gekennzeichnet, daß das Lösungsmittel N-Methylpyrrolidon ist.

16. Verfahren nach Anspruch 15, dadurch gekennzeichnet, daß das Lösungsmittel mindestens 40 Vol.-% des an der Reaktion teilnehmenden Alkohols ausmacht.

17. Verfahren nach einem der vorangegangenen Ansprüche, dadurch gekennzeichnet, daß der CO-Partialdruck weniger als 100 bar ausmacht.

18. Verfahren nach Anspruch 17, dadurch gekennzeichnet, daß der CO-Partialdruck 2 bis 60 bar beträgt.

FIG. 1